# EUROPÄISCHE PATENTSCHRIFT

(11) **EP 1 792 891 B1**
(45) Veröffentlichungstag und Bekanntmachung des Hinweises auf die Patenterteilung: **16.06.2010**
(21) Anmeldenummer: 06023304.6
(22) Anmeldetag: 09.11.2006
(51) Int. Cl.: C07C 201/02, C07C 203/04

(54) **Verfahren zur Herstellung von Salpetersäureestern einwertiger Alkohole**
Process for the production of nitric acid esters of monovalent alcohols
Procédé de fabrication d'esters d'acide nitrique des monoalcools

(30) Priorität: 21.11.2005 DE 102005055794; 30.11.2005 DE 102005056974; 30.11.2005 DE 102005057555
(43) Veröffentlichungstag der Anmeldung: 06.06.2007
(73) Patentinhaber: Josef Meissner GmbH & Co. KG, 50968 Köln (DE)
(72) Erfinder: Pöhlmann, Jürgen, Dr., 50969 Köln (DE); Pottharst, Frank, 50226 Frechen (DE); Hermann, Heinrich, Dr., 50858 Köln (DE); Konieczny, Peter, Dr., 14513 Teltow (DE); Händel, Mirko, Dr., 53819 Neunkirchen-Seelscheid (DE); Gebauer, Jürgen, 53840 Troisdorf (DE)
(74) Vertreter: Gesthuysen, von Rohr & Eggert

(56) Entgegenhaltungen:
- EP-A2- 0 129 995
- DE-A1- 2 039 609

## Beschreibung

Die vorliegende Erfindung betrifft ein Verfahren zur Herstellung von Salpetersäureestern (Nitratestern) einwertiger Alkohole.

Die Cetan-Zahl (kurz auch als CZ oder CaZ bezeichnet) ist eine der Octan-Zahl (OZ) analoge Kennziffer für die Zündwilligkeit eines Dieselkraftstoffs, wobei die Cetan-Zahl um so größer ist, je kürzer die Zeit (Zündverzug) zwischen Kraftstoffzuführung in den Motorzylinder und der Entzündung ist (vgl. Römpp Chemielexikon, 10. Auflage, Georg Thieme Verlag Stuttgart/New York, Stichwort: "Cetan-Zahl"). Als Cetan-Zahl-Verbesserer für Dieselkraftstoffe werden bereits seit vielen Jahren Nitratester von einwertigen Alkoholen, wie z. B. Amylnitrat, Hexylnitrat, Octylnitrat etc. und Isomeren davon, wie z. B. 2-Ethylhexylnitrat, eingesetzt.

Obwohl die Mononitrate dieser Alkohole selbst nicht als Explosivstoffe eingestuft und recht stabil sind, ist ihre Herstellung mit Risiken behaftet. Die Abfallsäuren aus der Nitrierung sind zum Teil nicht stabil, sondern können sich unkontrolliert zersetzen. Außerdem kann es zu heftigen oxidativen Zersetzungen des Produktes im Verlauf der Nitrierung kommen, verbunden mit einem sogenannten "Abrauchen" oder "Durchgehen der Reaktion" bzw. "Fume-off" oder sogar eine Explosion (vgl. z. B. Health Hazard Evaluation Report No. HETA 82-285-1339, referiert in Chem. Abstracts 102, 190181 (1985)).

Es wurden daher verschiedene Vorschläge gemacht, diese Risiken zu minimieren bzw. zu vermeiden.

So wird in der US-A-2 768 964 vorgeschlagen, die Veresterung einwertiger Alkohole kontinuierlich isotherm mit Mischsäuren aus Schwefelsäure und Salpetersäure mit einem Wassergehalt von 30 bis 50 % in Gegenwart von Harnstoff (1 bis 10 %) bei Temperaturen von 65 bis 110 °C im Vakuum durchzuführen. Die gebildeten Nitroester werden destillativ aus dem Reaktionsgemisch entfernt. Durch diese Verfahrensweise soll eine durch oxidative Nebenreaktionen bedingte, unkontrollierte Zersetzung durch die allgegenwärtige Nitrose unterdrückt werden.

Dieses Verfahren ist jedoch aufwendig und liefert mit längerkettigen Alkoholen, wie z. B. Octanol, nur Ausbeuten von maximal 52 %. Außerdem führt der hohe Wassergehalt in den Nitriersäuren zu instabilen Nitriersäuren.

Wird die Veresterung ohne Harnstoff kontinuierlich isotherm im Rührkessel oder einer Rührkesselkaskade durchgeführt, wird im Stand der Technik üblicherweise mit Mischsäuren aus Salpetersäure und Schwefelsäure mit einem Wassergehalt von 0 bis 14 % bei möglichst tiefen Temperaturen von -15 bis max. 20 °C, bevorzugt unterhalb von 10 °C, in Gegenwart eines Überschusses von ca. 5 % Salpetersäure, bezogen auf den zu nitrierenden Alkohol, gearbeitet (vgl. US-Patente 2 618 650, 2 734 910 und 4 479 905).

Das Gewichtsverhältnis von Wasser zu Schwefelsäure in der Nitrierendsäure sollte dabei nicht größer 0,35 sein, um die Gefahr eines "Fume-off" möglichst zu vermeiden (vgl. US-A-2 734 910).

Weiterhin sollten die Verweilzeiten für das Reaktionsgemisch in den Reaktoren möglichst kurz sein, insbesondere 0,6 bis 15 Minuten, bevorzugt 3 bis 6 Minuten, um eine Anreicherung von Nebenprodukten aus oxidativen Nebenreaktionen möglichst zu vermeiden.

Nur die Kombination der vorgenannten Parameter, nämlich tiefe Temperaturen bei der Nitrierung, möglichst kurze Verweilzeiten des Reaktionsgemisches in den Reaktoren und ein in der Nitrierendsäure vorliegendes Gewichtsverhältnis von Wasser zu Schwefelsäure unterhalb von 0,35, erlaubt nach dem Stand der Technik eine relativ sichere, kontinuierliche isotherme Veresterung von primären und sekundären Alkoholen mit Salpetersäure.

Auch diese Verfahren sind aufwendig und erfordern einen hohen Aufwand an Überwachung.

Die DE 2 039 609 A1 betrifft ein Verfahren zur Herstellung von Alkylnitraten durch isotherme Umsetzung eines entsprechenden Alkohols mit einem nitrierenden Säuregemisch, welches Salpetersäure, eine andere, hauptsächlich als Entwässerungsmittel wirkende Säure sowie ein Stabilisierungsmittel, insbesondere Harnstoff, enthält, derart, daß ein Gemisch aus Alkylnitrat und verbrauchter Säure entsteht, gefolgt von einer Abtrennung des auf diese Weise hergestellten Alkylnitrats von der verbrauchten Säure, beispielsweise durch Schwerkrafttrennung, Verdünnung mit Wasser oder Lösungsmittelextraktion, wobei die zu Zwecken der Nitrierung eingesetzte Nitriersäure aus Salpetersäure in Mengen von 0,8 bis 1,5 mol und Schwefelsäure als Entwässerungsmittel in Mengen von 1,5 bis 4,0 mol pro mol des Alkohols in Gegenwart von etwa 0,2 bis 2,0 Gew.-% des Alkohols und eines Produktstabilisierungsmittels, wie Harnstoff, gebildet wird und die Reaktion bei Temperaturen im Bereich zwischen 15°C und 0 °C isotherm durchgeführt wird, wobei Ausbeuten von 75 % und mehr erhalten werden sollen.

Das der vorliegenden Erfindung zugrundeliegende Problem liegt somit in der Bereitstellung eines Verfahren zur Herstellung von Salpetersäureestern (synonym auch Nitratester bzw. Nitroester genannt) einwertiger Alkohole, welches die zuvor geschilderten Nachteile bzw. Probleme des Standes der Technik zumindest weitgehend vermeidet oder aber wenigstens abschwächt.

Die Anmelderin hat nun überraschend herausgefunden, daß das zuvor geschilderte Problem dadurch gelöst werden kann, daß man das Verfahren derart durchführt, daß die einwertigen Alkohole mit einer Salpetersäure/Schwefelsäure-Mischsäure ("Nitriersäure") unter adiabatischen Reaktionsbedingungen kontinuierlich umgesetzt werden.

Zur Lösung des zuvor geschilderten Problems schlägt die vorliegende Erfindung somit ein Verfahren nach Patentanspruch 1 vor. Weitere, vorteilhafte Ausgestaltungen sind Gegenstand der betreffenden Verfahrensunteransprüche.

Gegenstand der vorliegenden Erfindung ist somit ein Verfahren zur Herstellung von Salpetersäureestern (Nitratestern) einwertiger Alkohole, wobei ein einwertiger Alkohol oder eine Mischung einwertiger Alkohole mit Salpetersäure in Gegenwart von Schwefelsäure unter adiabatischen Reaktionsbedingungen umgesetzt wird (Neben Salpetersäure und Schwefelsäure enthält das Veresterungsreagenz im allgemeinen auch Wasser, üblicherweise in variablen Mengen, d. h. im allgemeinen wird ein wäßriges Salpetersäure/Schwefelsäure-Gemisch als Veresterungsreagenz eingesetzt.). Mit anderen Worten wird nach dem erfindungsgemäßen Verfahren der einwertige Alkohol bzw. die Mischung einwertiger Alkohole adiabatisch mit einem Salpetersäure/Schwefelsäure-Gemisch ("Nitriersäure") bzw. mit einer Salpetersäure/Schwefelsäure-Mischsäure adiabatisch umgesetzt, so daß eine Veresterung eintritt. Im allgemeinen wird das erfindungsgemäße Verfahren kontinuierlich durchgeführt; grundsätzlich ist aber auch eine diskontinuierliche, d. h. chargenweise Durchführung möglich, obwohl der kontinuierliche Betrieb bevorzugt ist.

Im Gegensatz zu den Verfahren des Standes der Technik wird erfindungsgemäß die Umsetzung also nicht isotherm betrieben, sondern adiabatisch bzw. isentropisch, d. h. ohne Wärmeaustausch mit der Umgebung. Dabei kann die Umsetzung bei relativ hohen Temperaturen im Bereich von 10 bis 80 °C durchgeführt werden.

Denn im Rahmen der vorliegenden Erfindung hat es sich nun überraschenderweise gezeigt, daß die Veresterung von einwertigen Alkoholen, wie z. B. Amylalkoholen, Hexanol, Heptanol, Octanol etc. und deren Isomeren, mit einer Mischsäure aus Schwefelsäure und Salpetersäure kontinuierlich nicht nur - wie im Stand der Technik vorgeschlagen - isotherm bei tiefen Temperaturen möglich ist, sondern erfindungsgemäß auch adiabatisch, insbesondere in einem Temperaturbereich von 10 bis 80 °C, bevorzugt 10 bis 70 °C, besonders bevorzugt 20 bis 60 °C, mit hohen Ausbeuten, vorzugsweise in einem Rohrreaktor, sicher durchgeführt werden kann.

Adiabatische Umsetzungen mit Mischsäuren aus Salpetersäure und Schwefelsäure sind grundsätzlich aus dem Nitroaromatenbereich bereits bekannt und werden z. B. zur Herstellung von Nitrobenzol eingesetzt (vgl. US-Patente 4 021 498 und 4 091 042). Bislang sind adiabatische Umsetzung zur Herstellung von Salpetersäureestern jedoch im Stand der Technik nicht in Betracht gezogen worden.

Überraschend ist insbesondere, daß die Umsetzung des bzw, der einwertigen Alkohole nur mit einer Mischsäure aus Salpetersäure und Schwefelsäure erfolgt, d. h. zusätzlich keine anderen Reagenzien, insbesondere kein Harnstoff, vorgesehen ist.

Im Rahmen der vorliegenden Erfindung werden als einwertige Alkohole bevorzugt primäre einwertige Alkohole eingesetzt. Grundsätzlich ist aber auch die Umsetzung sekundärer und tertiärer einwertiger Alkohole möglich. Im Rahmen der vorliegenden Erfindung werden als einwertige Alkohole, vorzugsweise als primäre einwertige Alkohole, C₄- bis C₁₂-Alkohole, bevorzugt C₅- bis C₈-Alkohole, eingesetzt, vorzugsweise aus der Gruppe von Amylalkoholen, Hexanol, Heptanol und Octanol sowie deren Isomeren und Gemischen.

Wie zuvor beschrieben, wird die Umsetzung bzw. Veresterung insbesondere in einem Temperaturbereich von 10 bis 80 °C, bevorzugt 10 bis 70 °C, besonders bevorzugt 20 bis 60 °C, durchgeführt.

In erfindungsgemäß vorteilhafter Weise wird die Umsetzung in einem Reaktor durchgeführt. Dabei beträgt die Umsetzungsdauer (Reaktionsdauer), d. h. die gesamte Verweilzeit im Reaktor, insbesondere 0,01 bis 30 Sekunden, bevorzugt 0,1 bis 20 Sekunden, besonders bevorzugt 0,1 bis 10 Sekunden. Für die Umsetzung besonders bewährt hat sich insbesondere ein Rohrreaktor; ein derartiger Rohrreaktor sollte mindestens eine Mischeinrichtung und mindestens eine Verweilzeitstrecke, insbesondere ein Verweilzeitrohr, umfassen, wobei im Rohrreaktor, insbesondere im Verweilzeitrohr, noch zusätzliche Mischelemente angeordnet sein können,

Um einen effizienten Verfahrensablauf zu gewährleisten, sollte bei der Unsetzung bzw. Veresterung eine Mischenergie von 10 bis 1000 J/l (Joule/Liter), bevorzugt 10 bis 300 J/l, besonders bevorzugt 10 bis 100 J/l, in das Reaktionsgemisch eingebracht werden.

Das nach der Umsetzung resultierende Gemisch aus Nitrierendsäure und Produkt (Salpetersäureester bzw. Nitratester) wird im allgemeinen einer Phasentrennung unterworfen. Dabei ist es vorteilhaft, wenn das Gemisch aus Nitrierendsäure und Produkt nach Verlassen des Reaktors und vor Phasentrennung zunächst auf Temperaturen von 10 bis 30 °C, bevorzugt 15 bis 20 °C, abgekühlt wird.

Um einen effizienten Reaktionsablauf zu gewährleisten, sollte das Mischungsverhältnis von Salpetersäure zu Schwefelsäure in der Ausgangsmischung und/ oder die Ausgangskonzentrationen der eingesetzten Säuren in der Ausgangsmischung derart gewählt werden, daß das Masseverhältnis von Schwefelsäure zu Wasser in der Nitrierendsäure (d. h. in der Säure, die nach Reaktionsablauf vorliegt) mindestens 2 : 1 beträgt und insbesondere im Bereich von 2; 1 bis 5 : 1, bevorzugt im Bereich von 3 : 1 bis 4,5 : 1, liegt.

Ferner sollte zur Gewährleistung eines effizienten Reaktionsablaufs das Mischungsverhältnis von Salpetersäure zu Schwefelsäure in der Ausgangsmischung derart gewählt werden, daß die nach Umsetzung resultierende Nitrierendsäure einen Restgehalt von mindestens 0,5 Gew.-%, bevorzugt von mindestens 1 Gew.-%, besonders bevorzugt im Bereich von 1 bis 4 Gew.-%, Salpetersäure aufweist. Dies bedeutet mit anderen Worten, daß mit einem stöchiometrischen Überschuß an Salpetersäure in bezug auf die zu veresternden Alkohole verfahren wird.

Im allgemeinen kann als Umsetzungs- bzw. Veresterungsreagenz ein Salpetersäure/Schwefelsäure-Gemisch, welches variable Mengen an Wasser enthält, eingesetzt werden, insbesondere ein Gemisch auf Basis einer 50- bis 99%igen, vorzugsweise 65- bis 99%igen Salpetersäure zusammen mit einer 80- bis 99%igen, insbesondere 85- bis 96%igen Schwefelsäure und gegebenenfalls einem gewissen Anteil an rezyklierter Nitrierendsäure (wobei die rezyklierte Nitrierendsäure insbesondere die zuvor angegebene Zusammensetzung aufweisen kann).

Ein Teil der Nitrierendsäure kann wieder zur Herstellung der Ausgangsnitriersäure zurückgeführt werden. Alternativ braucht aber auch keine Nitrierendsäure zur Herstellung der Ausgangsnitrielsäure verwendet zu werden.

Erfindungsgemäß werden die zu veresternden einwertigen Alkohole zu Zwec??ken der Reaktion zusammen mit der Misch- bzw. Nitriersäure in eine Mischzone und nachfolgend in eine rohrförmige Reaktionszone eingebracht, in welcher die Umsetzung dann vollständig erfolgt. Gegebenenfalls können noch weitere Mischelemente im Reaktionsrohr derart angeordnet sein, daß eine optimale Verteilung des Gemisches von Nitratester und Alkohol in der Nitriersäure über die gesamte Strecke des Reaktionsrohres erfolgt.

Die Reaktionspartner Nitriersäure und zu veresternder Alkohol können z. B. mit insbesondere pulsationsarmen Dosierpumpensystemen im vorgesehenen Masseverhältnis Nitriersäure/Alkohol in den Reaktor derart eingespeist werden, daß die notwendige Mischenergie eingebracht werden kann.

Durch das erfindungsgemäße Verfahren wird ein Umsatz des zu veresternden Alkohols zu Nitratester von mehr als 99 %, vorzugsweise von mindestens 99,5 %, erreicht. Weitere Vorteile des Verfahrens sind eine Minimierung des Reaktorvolumens und damit des sogenannten "Hold-up" im Reaktor, verbunden mit kurzen Anfahrzeiten und sekundenschnellem Stop- bzw. Abbruchmöglichkeiten bei Unregelmäßigkeiten im Reaktionsablauf.

In Verbindung mit einer schnellen und effektiven Phasentrennung wird damit eine Anreicherung von Nebenprodukten in der Nitrierendsäure weitestgehend minimiert.

Der Reaktor, welcher mindestens eine Mischzone und mindestens eine rohrförmige Reaktions- bzw. Verweilzone umfaßt, wobei die Mischzone(n) und die Reaktions- bzw. Verweilzone(n) gegebenenfalls zusammenfallen können, wird im allgemeinen nicht gekühlt. Dadurch steigt die Temperatur im Reaktionsgemisch von der Temperatur der Mischsäure und des zu veresternden Alkohols durch die freigesetzte Reaktionswärme und die Verdünnungswärme der Nitriersäure auf die festgesetzte Endtemperatur an. Die Nitriersäure dient dabei als Energiespeicher für die im Verlauf der Umsetzung freigesetzten Wärmemenge.

Die Endtemperatur im Reaktionsgemisch ergibt sich aus der Eingangstemperatur der Reaktionspartner Nitriersäure und Alkohol und dem Verhältnis Nitriersäure/Alkohol und kann damit präzise auf die Eigenschaften des zu veresternden Alkohols und der Reaktionsmischung angepaßt werden.

Im Vergleich zu einer Veresterung des einwertigen Alkohols bei kontinuierlicher isothermer Betriebsweise z. B. in einem Rührkessel kann bei dem erfindungsgemäßen Verfahren durch die adiabatische Umsetzung und die damit verbundene Temperaturerhöhung um 20 bis 50 °C am Ende der Reaktion z. B. in einem Rohrreaktor nicht nur die Umsetzung der Restmengen an noch nicht umgesetztem Alkohol im Reaktionsgemisch beschleunigt, sondern auch die Verweilzeit des Veresterungsgemisehes aus Mischsäure und Alkohol im Reaktor wesentlich verkürzt werden. Die Verweilzeiten betragen bei der erfindungsgemäßen Arbeitsweise- je nach zu veresterndem Alkohol und eingesetztem Mischer - im allgemeinen nur 0,01 bis 30 Sekunden, bevorzugt 0,1 bis 20 Sekunden, besonders bevorzugt 0,1 bis 10 Sekunden.

In der Mischzone wird - insbesondere wenn Alkohole verestert werden sollen, die nur eine geringe Löslichkeit in der Nitriersäure besitzen- der zu verestemde Alkohol derart in der Nitriersäure verteilt, daß eine optimale Veresterung des umzusetzenden Alkohols möglich ist.

Die notwendige Vermischung der Reaktionspartner kann entweder durch passive Mischelemente oder aber durch anderweitiges zusätzliches Einbringen von Mischenergie in das Reaktionsgemisch erfolgen.

Als Mischorgane können z. B. Y-Mischer, statische Mischer, Blenden etc. eingesetzt werden. Die insgesamt eingebrachte Mischenergie sollte im Bereich von 10 bis 1000 J/l (Joule/Liter), bevorzugt 10 bis 200 J/l, besonders bevorzugt 10 bis 100 J/l, liegen.

Wird zu wenig Mischenergie in das Reaktionsgemisch eingebracht, so daß eine nichtoptimale Verteilung der schwerlöslichen Organphase in der Säurephase vorliegt, resultiert nicht nur eine unvollständige Umsetzung des zu veresternden Alkohols in der vorgesehenen Verweilzeit, sondern es besteht die zusätzliche Gefahr der Bildung von reaktiven Nebenprodukten durch oxidative Nebenreaktionen des eingesetzten Alkohols mit der überschüssigen Salpetersäure, verbunden mit dem Risiko eines sogenannten Durchgehens der Reaktion ("Fume-off").

Nach beendigter Reaktion erfolgt die Phasentrennung des Salpetersäureesters (Nitratester bzw. Nitroester) von der Nitrierendsäure. Dafür sollte das Nitriergemisch soweit abgekühlt werden, daß die Löslichkeit des Nitratesters in der Nitrierendsäure derart reduziert wird, daß eine sichere Lagerung der Endsäure ohne die Gefahr des Nachscheidens möglich ist, d. h. ohne die Gefahr der Bildung von Zwei-Phasen-Gemischen im Säurelager bei Überschreiten der Löslichkeitsgrenze des veresterten Alkohols in der Nitrierendsäure beim Abkühlen. Dies kann beispielsweise erreicht werden, indem der Reaktionszone aus Mischer und Nachreaktionszone unmittelbar eine Kühlzone nachgeschaltet wird, in der das Nitriergemisch auf 10 bis 30 °C, bevorzugt 15 bis 20 °C, abgekühlt wird.

Die abgetrennte und gekühlte Nitrierendsäure kann zusammen mit der frischen Mischsäure wieder in den Prozeß zurückgeführt werden, um das gewünschte Masseverhältnis von Nitriersäure zu zu nitrierendem Alkohol einzustellen, damit die gewählte Endtemperatur der adiabatischen Umsetzung nicht überschritten wird. Mit anderen Worten kann die Menge der rückgeführten Nitrierendsäure derart gewählt werden, daß die gewünschte Endtemperatur erreicht wird.

Die Zusammensetzung der Nitriersäure am Ende der Umsetzung wird derart gewählt, daß sowohl die Löslichkeit des veresterten Alkohols in der Nitriersäure als auch die Gefahr einer oxidativen Zersetzung von in der Nitrierendsäure gelösten Nebenprodukten möglichst gering ist.

Beispielsweise kann eine Nitriersäure aus rückgeführter Nitrierendsäure, konzentrierter Schwefelsäure und Salpetersäure und/oder Gemischen daraus ("Mischsäure") eingesetzt werden, die nach der Veresterungsreaktion des eingesetzten einwertigen Alkohols (d. h. in der Nitrierendsäure) ein Masseverhältnis von Schwefelsäure/Wasser von mindestens 2 : 1, insbesondere im Bereich von 2 : 1 bis 5 : 1, bevorzugt im Bereich von 3 : 1 bis 4,5 : 1, und/oder eine Salpetersäurekonzentration nach der Reaktion (d. h. in der Nitrierendsäure) von mindestens 0,5 % (Gew.-%), bevorzugt mindestens 1 %, vorzugsweise im Bereich von 1 bis 4 %, aufweist.

Neben der rückgeführten Nitrierendsäure kann die für die Veresterung von einwertigen Alkoholen eingesetzte Mischsäure aus beliebigen Ausgangssäuren hergestellt werden, sofern die vorgegebene Zusammensetzung der Nitrierendsäure nach erfolgter Umsetzung theoretisch erreichbar ist. So kann durch Zusatz von z. B. 96%iger Schwefelsäure mit 65%iger Salpetersäure oder 96%iger Schwefelsäure mit 99%iger Salpetersäure oder 85%iger Schwefelsäure mit 98%iger Salpetersäure oder Gemischen daraus ("Mischsäure") zu der rückgeführten Nitrierendsäure eine Nitriersäure hergestellt werden, aus der nach beendeter Veresterung die vorgelegte Nitrierendsäure erhalten wird.

Die zum Einsatz gelangenden Schwefel- und Salpetersäuren sind nicht auf die zuvor angegebene Konzentrationen beschränkt Außerdem kann, auf die Rückführung von Nitrierendsäure verzichtet werden, solange es die zum Einsatz gebrachte Menge Mischsäure erlaubt, die gesamte freigesetzte Wärmemenge aus der Veresterung und Verdünnung der Mischsäure derart aufzunehmen, daß die festgesetzte Endtemperatur am Ende der adiabatischen Umsetzung nicht überschritten wird.

Die Phasentrennung des Reaktionsgemisches in Produkt und Säurephase kann in statischen Scheidern oder aber in dynamischen Separatoren erfolgen, wobei der Einsatz von dynamischen Separatoren bevorzugt wird, um die Kontaktzeit der Nitrierendsäure mit dem Produkt und damit die Gefahr der Anreicherung von Zersetzungsprodukten aus oxidativen Nebenreaktionen mit Salpetersäure in der Säure- und der Organphase zu minimieren.

Der von der Nitrierendsäure abgetrennte Salpetersäure- bzw. Nitroester wird - wie üblich - in drei Stufen zuerst mit Wasser, nachfolgend mit einer Alkalilösung und dann noch einmal mit Wasser gewaschen. Das Waschwasser aus der dritten Waschstufe wird vorteilhaft in der ersten Waschstufe zur Abtrennung der überschüssigen Säure eingesetzt.

Die Trennung der Waschemulsion nach jeder Waschstufe kann im statischen Scheider oder mit dynamischen Separatoren durchgeführt werden.

Die abgetrennte Nitrierendsäure kann zumindest teilweise im Kreis geführt werden. Die Nitrierendsäure bzw. im Fall der Kreisführung der Überschuß an Nitrierendsäure kann in einer SAC-Anlage (SAC = Sulphuric Acid Concentration) derart aufbereitet und aufkonzentriert werden, daß sie wieder in die Nitrierung zurückgeführt werden kann.

Bei dem nach dem erfindungsgemäßen Verfahren herstellbaren Salpetersäureester kann es sich grundsätzlich um alle aus einwertigen Alkoholen herstellbare Salpetersäureester handeln, bevorzugt aber um Salpetersäureester primärer einwertiger Alkohole, die bei 0 °C flüssig sind und zu flüssigen Nitratestern führen.

Das Verfahren ist weiter nicht beschränkt auf einwertige Alkohole, die mit der Nitriersäure mischbar sind bzw. eine noch gute Löslichkeit in der Nitriersäure besitzen, sondern das Verfahren ist besonders geeignet auch für Alkohole mit schlechter Mischbarkeit und geringer Löslichkeit in der Mischsäure bzw. Endsäure, wie z. B. 2-Ethylhexan-1-ol, bei dem der Vorteil des beschriebenen Verfahrens beispielhaft gezeigt werden soll, ohne daß damit irgendwelche Einschränkungen verbunden sind.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sie jedoch hierauf zu beschränken.

### AUSFÜHRUNGSBEISPIELE:

### Beispiel 1: Nitrierung mit Mischsäure ohne Rückführung von Nitrierendsäure

Mit einem pulsationsarmen Pumpensystem wurden 1,563 kg/h 2-Ethylhexan-1-ol und 4,567 kg/h Mischsäure der Zusammensetzung 69,2 % Schwefelsäure, 18,2 % Salpetersäure und 12,6 % Wasser, hergestellt aus 85%iger Schwefelsäure und 98%iger Salpetersäure, über ein T-Rohr in den Reaktor, bestehend aus einem statischen Mischer und einer Reaktionszone, eingespeist.

Beide Dosierströme wurden auf 20 °C temperiert. Die Verweilzeit im Reaktor betrug insgesamt 4,0 Sekunden. Der spezifische Energieeintrag betrug ca. 64 J/1. Am Ende des Reaktors wurde eine Temperatur von 51,5 °C angezeigt. Die adiabatische Temperaturerhöhung betrug 31,5 °C. Unmittelbar nach dem Reaktor durchlief das Reaktionsgemisch ein Kühlbad und wurde auf 20 °C abgekühlt. Nach Phasentrennung wurden ca. 2.090 g des Salpetersäureesters von 2-Ethylhexan-1-ol erhalten (Rohausbeute ca. 99 %). Die Nitrierendsäure von ca. 4,0 kg/h hatte eine Zusammensetzung von 78,45 % Schwefelsäure, 1,85 % Salpetersäure und 19,7 % Wasser. Das Verhältnis Schwefelsäure zu Wasser betrug 3,98 : 1. Nach der üblichen dreistufigen Wäsche mit Wasser, Alkali und Wasser wurde ein Produkt mit einem Gehalt von 99,6 % des Salpetersäureesters von 2-Ethylhexan-1-ol erhalten, das noch 0,31 % Verunreinigungen, vorwiegend 2-Ethylhexan-1-ol und sonstige Nebenprodukte, enthielt.

### Beispiel 2: Nitrierung mit Mischsäure und Rückführung von Nitrierendsäure

Mit einem pulsationsarmen Pumpensystem wurden ca. 0,73 kg Mischsäure der Zusammensetzung 54,5 % Schwefelsäure, 43,5 % Salpetersäure und 2,0 % Wasser, hergestellt aus 97,2%iger Schwefelsäure und 99%iger Salpetersäure, mit ca. 2,01 kg/h Nitrierendsäure kontinuierlich gemischt.

Zu der resultierenden Nitriersäure mit der Zusammensetzung 71,5 % Schwefelsäure, 13,3 % Salpetersäure und 15,2 % Wasser wurden 630 g/h 2-Ethylhexan-1-ol über ein T-Rohr in den Reaktor, bestehend aus einem statischen Mischer und einer Reaktionszone, eingespeist.

Sowohl die Nitriersäure als auch das 2-Ethylhexan-1-ol wurden vor Vermischung auf ca. 20 °C gekühlt. Die Verweilzeit im Reaktor betrug insgesamt 8,0 Sekunden. Der spezifische Energieeintrag betrug ca. 36 J/l. Am Ende des Reaktors wurde eine Temperatur von 45,2 °C angezeigt. Die adiabatische Temperaturerhöhung betrug 25,2 °C. Unmittelbar nach dem Reaktor durchlief das Reaktionsgemisch ein Kühlbad und wurde auf 20 °C abgekühlt. Nach Phasentrennung wurden ca. 840 g des Salpetersäureesters von 2-Ethylhexan-1-ol erhalten (Rohausbeute ca. 99 %). Die Nitrierendsäure mit ca. 2,7 kg/h hatte eine Zusammensetzung von 77,6 % Schwefelsäure, 2,4 % Salpetersäure und 20,0 % Wasser. Das Verhältnis Schwefelsäure zu Wasser betrug 3,8 : 1. Nach der üblichen dreistufigen Wäsche mit Wasser, Alkali und Wasser wurde ein Produkt mit einem Gehalt von 99,5 % des Salpetersäureesters von 2-Ethylhexan-1-ol erhalten, das noch 0,4 % Verunreinigungen, vorwiegend 2-Ethylhexan-1-ol und sonstige Nebenprodukte, enthielt.

## Patentansprüche

1. Verfahren zur Herstellung von Salpetersäureestern (Nitratestern) einwertiger Alkohole,
**dadurch gekennzeichnet,**
**daß** ein einwertiger Alkohol oder eine Mischung einwertiger Alkohole mit Salpetersäure in Gegenwart von Schwefelsäure unter adiabatischen Reaktionsbedingungen umgesetzt wird.

2. Verfahren nach Anspruch 1, **dadurch gekennzeichnet, daß** als einwertige Alkohole bevorzugt primäre einwertige Alkohole eingesetzt werden und/oder daß als einwertige Alkohole, vorzugsweise primäre einwertige Alkohole, C₄- bis C₁₂-Alkohole, bevorzugt C₅- bis C₈-Alkohole, insbesondere aus der Gruppe von Amylalkoholen, Hexanol, Heptanol und Octanol sowie deren Isomeren und Gemischen, eingesetzt werden.

3. Verfahren nach Anspruch 1 oder 2, **dadurch gekennzeichnet, daß** die Umsetzung in einen Temperaturbereich von 10 bis 80°C, bevorzugt 10 bis 70 °C, besonders bevorzugt 20 bis 60 °C, durchgeführt wird und/oder daß die Umsetzung kontinuierlich durchgeführt wird.

4. Verfahren nach einem oder mehreren der vorangehenden Ansprüche, **dadurch gekennzeichnet, daß** die Umsetzung in einem Reaktor durchgeführt wird, insbesondere wobei die Umsetzungsdauer (Reaktionsdauer), insbesondere die gesamte Verweilzeit im Reaktor, 0,01 bis 30 Sekunden, bevorzugt 0,1 bis 20 Sekunden, besonders bevorzugt 0,1 bis 10 Sekunden, beträgt.

5. Verfahren nach einem oder mehreren der vorangehenden Ansprüche, **dadurch gekennzeichnet, daß** die Umsetzung in einem Rohrreaktor durchgeführt wird, insbesondere wobei der Rohrreaktor mindestens eine Mischeinrichtung und mindestens eine Venveilzeitstrecke, insbesondere ein Verweilzeitrohr, umfaßt und/oder daß im Rohrreaktor, insbesondere im Verweilzeitrohr, noch zusätzliche Mischelemente angeordnet sind.

6. Verfahren nach einem oder mehreren der vorangehenden Ansprüche, **dadurch gekennzeichnet, daß** insgesamt eine Mischenergie von 10 bis 1000 J/l (Joule/Liter), bevorzugt 10 bis 300 J/l, besonders bevorzugt 10 bis 100 J/l, in das Reaktionsgemisch eingebracht wird und/oder daß das nach der Umsetzung resultierende Gemisch aus Nitrierendsäure und Produkt einer Phasentrennung unterworfen wird, insbesondere wobei das Gemisch aus Nitrierendsäure und Produkt nach Verlassen des Reaktors und vor Phasentrennung zunächst auf Temperaturen von 10 bis 30 °C, bevorzugt 15 bis 20 °C, abgekühlt wird.

7. Verfahren nach einem oder mehreren der vorangehenden Ansprüche, **dadurch gekennzeichnet, daß** das Mischungsverhältnis von Salpetersäure zu Schwefelsäure in der Ausgangsmischung und/oder die Konzentration der eingesetzten Säuren in der Ausgangsmischung derart gewählt wird, daß das Masseverhältnis von Schwefelsäure zu Wasser in der Nitrierendsäure mindestens 2 : 1 beträgt und vorzugsweise im Bereich von 2 : 1 bis 5 : 1, bevorzugt im Bereich von 3 : 1 bis 4,5 : 1, liegt, und/oder daß das Mischungsverhältnis von Salpetersäure zu Schwefelsäure in der Ausgangsmischung derart gewählt wird, daß die nach Umsetzung resultierende Nitrierendsäure einen Restgehalt von mindestens 0,5 Gew.-%, bevorzugt mindestens 1 Gew.-%, besonders bevorzugt im Bereich von 1 bis 4 Gew.-%, Salpetersäure aufweiset.

8. Verfahren nach einem oder mehreren der vorangehenden Ansprüche, **dadurch gekennzeichnet, daß** ein Teil der Nitrierendsäure zur Herstellung der Ausgangsnitriersäure wieder zurückgeführt wird oder daß keine Nitrierendsäure zur Herstellung der Ausgangsnitriersäure verwendet wird.

9. Verfahren nach einem oder mehreren der vorangehenden Ansprüche, **dadurch gekennzeichnet, daß** die Umsetzung derart durchgeführt wird, daß die adiabatische Temperaturerhöhung, berechnet als Differenz der Temperatur zu Reaktionsbeginn und zu Reaktionsende, 20 bis 50 °C, insbesondere 25 bis 40 °C, beträgt.

10. Verfahren nach einem oder mehreren der vorangehenden Ansprüche, **dadurch gekennzeichnet, daß** als Veresterungsreagenz ein wäßriges Salpetersäure/Schwefelsäure-Gemisch eingesetzt wird, insbesondere wobei das wäßrige Salpetersäure/Schwefelsäure-Gemisch eine 80- bis 96%ige, vorzugsweise 85- bis 96%ige Schwefelsäure und/oder eine 50- bis 99%ige, vorzugsweise 65- bis 99%ige Salpetersäure und/oder gegebenenfalls rezyklierte Nitrierendsäure, insbesondere wie in Anspruch 7 definiert, umfaßt.

## Claims

1. Process for preparing nitric acid esters (nitrate esters) of monohydric alcohols, **characterized in that** a monohydric alcohol or a mixture of monohydric alcohols is reacted with nitric acid in the presence of sulphuric acid under adiabatic reaction conditions.

2. Process according to Claim 1, **characterized in that** primary monohydric alcohols are preferably used as monohydric alcohols and/or **in that** C₄-C₁₂-alcohols, preferably C₅-C₈-alcohols, in particular from the group consisting of amyl alcohols, hexanol, heptanol and octanol and also isomers and mixtures thereof, are used as monohydric alcohols, preferably primary monohydric alcohols.

3. Process according to Claim 1 or 2, **characterized in that** the reaction is carried out in the temperature range from 10 to 80 °C, preferably from 10 to 70 °C, particularly preferably from 20 to 60 °C, and/or **in that** the reaction is carried out continuously.

4. Process according to one or more of the preceding claims, **characterized in that** the reaction is carried out in a reactor, in particular with the reaction time, in particular the total residence time in the reactor, being from 0.01 to 30 seconds, preferably from 0.1 to 20 seconds, particularly preferably from 0,1 to 10 seconds.

5. Process according to one or more of the preceding claims, **characterized in that** the reaction is carried out in a tube reactor, in particular with the tube reactor comprising at least one mixing device and at least one residence section, in particular a residence tube, and/or **in that** additional mixing elements are arranged in the tube reactor, in particular in the residence tube.

6. Process according to one or more of the preceding claims, **characterized in that** a total mixing energy of from 10 to 1000 J/l (joule/litre), preferably from 10 to 300 J/l, particularly preferably from 10 to 100 J/l, is introduced into the reaction mixture and/or **in that** the mixture of residual nitrating acid and product resulting from the reaction is subjected to a phase separation, in particular with the mixture of residual nitrating acid and product firstly being cooled to temperatures of from 10 to 30 °C, preferably from 15 to 20 °C, after leaving the reactor and before phase separation.

7. Process according to one or more of the preceding claims, **characterized in that** the mixing ratio of nitric acid to sulphuric acid in the starting mixture and/or the concentration of the acids used in the starting mixture is selected so that the mass ratio of sulphuric acid to water in the residual nitrating acid is at least 2 : 1 and is preferably in the range from 2 : 1 to 5 : 1, more preferably in the range from 3 : 1 to 4.5 : 1, and/or **in that** the mixing ratio of nitric acid to sulphuric acid in the starting mixture is selected so that the residual nitrating acid resulting from the reaction has a residual content of at least 0.5 % by weight, preferably at least 1 % by weight, particularly preferably in the range from 1 to 4 % by weight, of nitric acid.

8. Process according to one or more of the preceding claims, **characterized in that** part of the residual nitrating acid is recirculated to the preparation of the starting nitrating acid or **in that** no residual nitrating acid is used for preparing the starting nitrating acid.

9. Process according to one or more of the preceding claims, **characterized in that** the reaction is carried out so that the adiabatic temperature increase, calculated as the difference between the temperature at the beginning of the reaction and at the end of the reaction, is from 20 to 50 °C, in particular from 25 to 40 °C.

10. Process according to one or more of the preceding claims, **characterized in that** an aqueous nitric acid/sulphuric acid mixture is used as esterification reagent, in particular with the aqueous nitric acid/sulphuric acid mixture comprising an 80-96 % strength, preferably 85-96 % strength, sulphuric acid and/or a 50-99 % strength, preferably 65-99 % strength, nitric acid and/or optionally recycled residual nitrating acid, in particular as defined in Claim 7.

## Revendications

1. Procédé pour la préparation d'esters d'acide nitrique (nitrates) avec des alcools monohydriques, **caractérisé en ce qu'**on fait réagir dans des conditions réactionnelles adiabatiques un alcool monohydrique ou un mélange d'alcools monohydriques avec de l'acide nitrique en présence d'acide sulfurique.

2. Procédé selon la revendication 1, **caractérisé en ce qu'**on utilise comme alcools monohydriques de préférence des alcools monohydriques primaires et/ou **en ce qu'**on utilise comme alcools monohydriques de préférence des alcools monohydriques primaires, des alcools en C₄-C₁₂, de préférence des alcools en C₅-C₈, en particulier choisis dans le groupe constitué par les alcools amyliques, l'hexanol, l'heptanol et l'octanol ainsi que leurs isomères et des mélanges de ces alcools.

3. Procédé selon la revendication 1 ou 2, **caractérisé en ce que** la réaction est effectuée dans une plage de température de 10 à 80 °C, de préférence de 10 à 70 °C, de façon particulièrement préférée de 20 à 60 °C et/ou **en ce que** la réaction est effectuée en continu.

4. Procédé selon une ou plusieurs des revendications précédentes, **caractérisé en ce que** la réaction est effectuée dans un réacteur, en particulier la durée de conversion (durée de réaction), en particulier le temps total de séjour dans le réacteur, allant de 0,01 à 30 secondes, de préférence de 0,1 à 20 secondes, de façon particulièrement préférée de 0,1 à 10 secondes.

5. Procédé selon une ou plusieurs des revendications précédentes, **caractérisé en ce que** la réaction est effectuée dans un réacteur tubulaire, en particulier le réacteur tubulaire comprenant au moins un dispositif doseur et au moins un segment de séjour, en particulier un tube de séjour et/ou **en ce que** des éléments mélangeurs supplémentaires sont encore disposés dans le réacteur tubulaire, en particulier dans le tube de séjour.

6. Procédé selon une ou plusieurs des revendications précédentes, **caractérisé en ce qu'**on introduit dans le mélange réactionnel au total une énergie de mélange de 10 à 1 000 J/l (joules/litre), de préférence de 10 à 300 J/l, de façon particulièrement préférée, de 10 à 100 J/l et/ou **en ce que** le mélange d'acide nitrant et de produit, résultant de la réaction, est soumis à une séparation de phases, en particulier le mélange d'acide nitrant et de produit est d'abord refroidi, après avoir quitté le réacteur et avant la séparation de phases, jusqu'à des températures de 10 à 30 °C, de préférence de 15 à 20 °C.

7. Procédé selon une ou plusieurs des revendications précédentes, **caractérisé en ce qu'**on choisit le rapport de mélange de l'acide nitrique à l'acide sulfurique dans le mélange de départ et/ou la concentration des acides utilisés dans le mélange de départ de manière que le rapport massique de l'acide sulfurique à l'eau dans l'acide nitrant soit d'au moins 2 : 1 et de préférence se situe dans la plage de 2 : 1 à 5 : 1, encore mieux dans la plage de 3 : 1 à 4,5 : 1, et/ou **en ce qu'**on choisit le rapport de mélange de l'acide nitrique à l'acide sulfurique dans le mélange de départ de manière que l'acide nitrant résultant après la réaction présente une teneur résiduelle en acide nitrique d'au moins 0,5 % en poids, de préférence d'au moins 1 % en poids, de façon particulièrement préférée dans la plage de 1 à 4 % en poids.

8. Procédé selon une ou plusieurs des revendications précédentes, **caractérisé en ce qu'**une partie de l'acide nitrant est renvoyée de nouveau à la préparation du mélange sulfonitrique de départ ou **en ce qu'**aucun acide nitrant n'est utilisé dans la préparation du mélange sulfonitrique de départ.

9. Procédé selon une ou plusieurs des revendications précédentes, **caractérisé en ce que** la réaction est effectuée de manière que l'élévation de température adiabatique, calculée en tant que différence de la température au début de la réaction et à la fin de la réaction, soit de 20 à 50 °C, en particulier de 25 à 40 °C.

10. Procédé selon une ou plusieurs des revendications précédentes, **caractérisé en ce qu'**on utilise comme réactif d'estérification un mélange aqueux d'acide nitrique/acide sulfurique, en particulier le mélange aqueux d'acide nitrique/acide sulfurique comprenant un acide sulfurique à 80-96 %, de préférence un acide sulfurique à 85-96 % et/ou un acide nitrique à 50-99 %, de préférence à 65-99 % et/ou éventuellement de l'acide nitrant recyclé, en particulier tel que défini dans la revendication 7.
